# EUROPÄISCHE PATENTANMELDUNG

(11) **EP 4 306 629 A1**
(43) Veröffentlichungstag der Anmeldung: **17.01.2024**
(21) Anmeldenummer: 23175585.1
(22) Anmeldetag: 26.05.2023
(51) Int. Cl.: C12M 1/00, C12M 1/09, C12M 1/12

(54) **ANORDNUNG UND VERFAHREN ZUR HERSTELLUNG EINER CELLULOSE-SCHICHTSTRUKTUR**

(30) Priorität: 16.06.2022 DE 102022206082
(71) Anmelder: VOLKSWAGEN AG, 38440 Wolfsburg (DE)
(72) Erfinder: Gottschling, Martina, 38518 Gifhorn (DE); Hauke, Sebastian, 61197 Florstadt (DE)

(57) **Zusammenfassung**

Die Erfindung betrifft eine Anordnung zur Herstellung einer Cellulose-Schichtstruktur, mit einem Behälter (5), cellulosebildende Mikroorganismen, einem Kulturmedium (11), einem Gas (17) und einem Celluloseträger (13), wobei der Behälter (5) zumindest teilweise mit dem Kulturmedium (11) befüllt ist, wobei der Celluloseträger (13) mit dem Kulturmedium (11) in Kontakt steht, und wobei die Anordnung (1) dazu ausgebildet ist, dass sich durch Wechselwirkung der Mikroorganismen, des Kulturmediums (11) und des Gases (17) an dem Celluloseträger (13) eine Cellulose-Schichtstruktur (15) bildet. Erfindungsgemäß ist der Celluloseträger (13) als ein relativ zum Behälter (5) beweglicher Celluloseträger (13) ausgebildet.

## Beschreibung

Die Erfindung betrifft eine Anordnung zur Herstellung einer Cellulose-Schichtstruktur nach dem Oberbegriff des Anspruches 1 sowie ein Verfahren zur Herstellung einer Cellulose-Schichtstruktur nach dem Anspruch 10.

Bei einer gattungsgemäßen Anordnung zur Herstellung einer Cellulose-Schichtstruktur aus bakterieller Nanocellulose erfolgt die Herstellung der Cellulose-Schichtstruktur in einem dynamischen Kultivierverfahren. Bei dem dynamischen Kultivierverfahren ist ein Behälter vorgesehen, der in einen Kreislauf eingebunden ist, in dem ein Kulturmedium zirkuliert, das Nährmedium enthält. Das zirkulierende Kulturmedium strömt kontinuierlich durch den Behälter. Im Bereich einer Grenzfläche zwischen dem Kulturmedium und einem in dem Behälter befindlichen, sauerstoffhaltigen Gas ist ein Celluloseträger angeordnet, der an dem Behälter befestigt ist. Auf dem Celluloseträger sind cellulosebildende Mikroorganismen angeordnet. Der Celluloseträger ist für das Kulturmedium durchlässig und für die Mikroorganismen undurchlässig. Das Kulturmedium steht im Bereich des Celluloseträgers in Kontakt mit dem Gas und das Gas wird von den Mikroorganismen unter Nutzung des Kulturmediums zu Cellulose verstoffwechselt, wobei sich eine auf dem Celluloseträger angeordnete Cellulose-Schichtstruktur bildet.

Um eine hohe mechanische Festigkeit der Cellulose-Schichtstruktur zu erreichen, ist eine Gasblasenbildung zwischen dem Celluloseträger und dem Kulturmedium sowie eine vollständige oder lokale Trennung einzelner Celluloseschichten voneinander zu vermeiden. Hierfür und um die Mikroorganismen prozesssicher mit ausreichend Nährstoffen zu versorgen, ist die Zuführung des Kulturmediums in und die Abführung des Kulturmediums aus dem Behälter mit einem hohen Steuer- und Regelungsaufwand verbunden. Ferner ist für die Aufrechterhaltung des Kreislaufes eine verhältnismäßig große Menge an Kulturmedium erforderlich, so dass es länger dauert bis sich in dem Kulturmedium ideale Wachstumsbedingungen für die cellulosebildenden Mikroorganismen einstellen.

Aus der WO 90/00595 A1 sind eine Vorrichtung und ein Verfahren für schwimmende Zellkulturen bekannt. Die DE 10 2005 002 938 A1 offenbart eine schwimmfähige Zell- oder Gewebeträgeranordnung sowie einen Perfusionscontainer.

Die Aufgabe der Erfindung besteht darin, eine Anordnung sowie ein Verfahren bereitzustellen, mit denen eine Cellulose-Schichtstruktur mit hoher mechanischer Festigkeit kostengünstig und zeiteffizient herstellbar ist.

Diese Aufgabe ist durch die Merkmale der Ansprüche 1, 9 oder 10 gelöst. Bevorzugte Weiterbildungen der Erfindung sind in den Unteransprüchen offenbart.

Die Erfindung geht von einer Anordnung zur Herstellung einer Cellulose-Schichtstruktur, vorzugsweise zur Herstellung einer bakteriellen Nanocellulose-Schichtstruktur, aus, mit einem Behälter, cellulose- oder nanocellulosebildenden Mikroorganismen, einem Kulturmedium, einem Gas, vorzugsweise Umgebungsluft, und einem Celluloseträger, wobei der Behälter zumindest teilweise mit dem Kulturmedium und vorzugsweise teilweise mit dem Gas befüllt ist, wobei der Celluloseträger mit dem Kulturmedium in Kontakt steht, und wobei die Anordnung dazu ausgebildet ist, dass sich, vorzugsweise durch Wechselwirkung der Mikroorganismen, des Kulturmediums und des Gases, an dem Celluloseträger eine Cellulose-Schichtstruktur oder eine bakterielle Nanocellulose-Schichtstruktur bildet. Erfindungsgemäß ist der Celluloseträger als ein beweglicher und/oder bewegbarer Celluloseträger ausgebildet, der relativ zum Behälter, vorzugsweise zu einer Behälterwand des Behälters, beweglich und/oder bewegbar ist.

Bevorzugt können die Mikroorganismen, das Kulturmedium, das Gas und der Celluloseträger in dem Behälter aufgenommen sein.

Der Celluloseträger kann bevorzugt zumindest teilweise, vorzugsweise ausschließlich, in Gleitverbindung mit dem Behälter, vorzugsweise der Behälterwand, stehen. Alternativ dazu kann der Celluloseträger außer Anlage mit dem Behälter, vorzugsweise der Behälterwand sein. Das kann heißen, dass der Celluloseträger vollständig oder zumindest teilweise außer Kontakt mit dem Behälter, vorzugsweise der Behälterwand, ist. Der Behälter kann einen Behälterboden aufweisen, wobei bevorzugt vorgesehen sein kann, dass der Behälter und/oder die Behälterwand dreh- oder rotationssymmetrisch zu einer Behälter-Hochachse ist. Alternativ kann der Behälter quader- oder würfelförmig sein, wobei vorzugsweise die Behälterwand rechteck- oder quadratrohrförmig ausgebildet sein kann. Der Celluloseträger kann, vorzugsweise ausschließlich, entlang der Behälter-Hochachse relativ zum Behälter beweglich und/oder bewegbar sein. Alternativ oder zusätzlich kann der Celluloseträger um die Behälter-Hochachse relativ zum Behälter beweglich, vorzugsweise verdrehbar, sein. Die Behälter-Hochachse kann eine nach oben zeigende, positive Achsenrichtung aufweisen, die entgegen der Lotrichtung gerichtet sein kann.

Die bewegliche Ausgestaltung des Celluloseträgers hat den Vorteil, dass die Position des Celluloseträgers an sich ändernde Bedingungen bei der Herstellung der Cellulose-Schichtstruktur anpassbar ist. Dies ermöglicht die Herstellung der Cellulose-Schichtstruktur bei gleichbleibend hoher Qualität auch in den Fällen, in denen sich beispielsweise die Menge des Kulturmediums im Behälter ändert.

In einer konkreten Ausführungsform kann die Cellulose-Schichtstruktur eine Nanocellulose-Schichtstruktur aus, bevorzugt bakterieller, Nanocellulose sein.

In einer bevorzugten Ausführungsform kann das Kulturmedium, vorzugsweise flüssiges, Nährmedium und/oder die cellulosebildenden Mikroorganismen aufweisen, wobei das Nährmedium und die cellulosebildenden Mikroorgansimen bevorzugt miteinander vermischt sein können. Die cellulosebildenden Mikroorganismen können aeroben Bakterien sein, die ausgewählt sein können aus der Gruppe aufweisend Komagataeibacter xylinus, Rhizobium leguminosarum, Agrobacterium tumefaciens, Escherichia coli, Gluconacetobacter xylinus oder Gemischen daraus oder Gemischen mit anderen Mikroorganismen wie z.B. Hefen.

In einer besonders bevorzugten Ausführungsform kann das Gas ein sauerstoffhaltiges Gas, vorzugsweise Umgebungsluft, sein. Die cellulosebildenden Mikroorganismen können sich auf dem Celluloseträger und/oder im Kontaktbereich von Kulturmedium und Gas anlagern und mit dem Sauerstoff des sauerstoffhaltigen Gases reagieren und unter Versorgung mit Nährstoffen aus dem Nährmedium Cellulose synthetisieren, und zwar unter Bildung der Cellulose-Schichtstruktur, vorzugsweise der Nanocellulose-Schichtstruktur.

Besonders bevorzugt bildet sich die Cellulose-Schichtstruktur selbstständig, das heißt vorzugsweise rein passiv und/oder vorzugsweise ohne ein aktives Aufbringen der cellulosebildenden Mikroorganismen auf den Celluloseträger, wie dies beispielsweise durch aktiven Impfens erfolgen könnte.

Bevorzugt kann die Cellulose-Schichtstruktur ein oder mehrere flächig ausgebildete Cellulose-Schichten aufweisen, die untereinander quervernetzt sein können. Die Cellulose-Schichten können sich vorzugsweise in der Horizontalen erstrecken und/oder parallel zueinander verlaufen. Vorzugsweise kann die Cellulose-Schichtstruktur gallertartig sein. Die Cellulose-Schichtstruktur kann bevorzugt dadurch gebildet werden, dass entgegen der Lotrichtung und auf dem Celluloseträger einzelne Cellulose-Schichten nacheinander gebildet werden, so dass die Cellulose-Schichtstruktur entgegen der Lotrichtung Schicht für Schicht wächst. Durch die Quervernetzung der Cellulose-Schichten ergibt sich eine mechanisch feste Cellulose-Schichtstruktur, die beispielsweise als Lederalternative oder Alternative zu erdölbasiertem Kunststoff einsetzbar ist.

Bevorzugt kann die Herstellung der Cellulose-Schichtstruktur in Standkultur in dem Behälter im einfachen Satzverfahren erfolgen. Die Cellulose-Schichtstruktur ist somit kostengünstig und bei geringem apparativem Aufwand herstellbar.

In einer konkreten Ausführungsform kann der Celluloseträger als ein auf dem Kulturmedium schwimmender Celluloseträger ausgebildet sein. Alternativ oder zusätzlich kann der Celluloseträger auf dem Kulturmedium schwimmen.

Insbesondere beim einfachen Satzverfahren verbraucht sich das Kulturmedium während sich die Cellulose-Schichtstruktur bildet und/oder wächst. Durch die schwimmende Ausgestaltung des Celluloseträgers ist gewährleistet, dass der Celluloseträger stets mit dem Kulturmedium in Kontakt steht und sich unter dem Celluloseträger keine unerwünschten Gasblasen bilden. So ist eine Cellulose-Schichtstruktur mit hoher mechanischer Festigkeit auch im einfachen Satzverfahren prozesssicher herstellbar.

In einer konkreten Ausführungsform kann der Celluloseträger durch ein, vorzugsweise kreis- oder rechteckscheibenförmiges, Membranelement und durch einen, vorzugsweise die Form einer Kreisringscheibe oder einer Rechteckringscheibe aufweisenden, Schwimmkörper gebildet sein. Bevorzugt kann vorgesehen sein, dass der Celluloseträger, vorzugsweise das Membranelement und/oder der Schwimmkörper, für das Kulturmedium, vorzugsweise für das Nährmedium und/oder für die cellulosebildenden Mikroorganismen, durchlässig ist.

Bevorzugt kann das Membranelement eine Membranelement-Oberseite und eine Membranelement-Unterseite aufweisen, wobei die Membranelement-Oberseite dem Behälterboden abgewandt und die Membranelement-Unterseite dem Behälterboden zugewandt sein kann.

Bevorzugt können das Membranelement und der Schwimmkörper voneinander separat hergestellte und vorzugsweise miteinander gefügte Einzelbauteile sein. Besonders bevorzugt kann der Schwimmkörper durch ein oder mehrere Schwimmkörperelemente gebildet sein. Bevorzugt kann der Schwimmkörper zumindest teilweise oder vollständig, vorzugsweise in Bezug auf die Behälter-Hochachse gesehen, an und/oder unter der Membranelement-Unterseite angeordnet sein.

In einer konkreten Ausführungsform kann der Schwimmkörper an dem Membranelement befestigt sein. Bevorzugt kann vorgesehen sein, dass das Membranelement an einer Schwimmkörper-Innenumfangsfläche des Schwimmkörpers befestigt ist. Alternativ kann der Schwimmkörper zumindest teilweise, vorzugsweise ausschließlich, an der Membranelement-Unterseite befestigt sein. Vorzugsweise kann der Schwimmkörper aus einem geschäumten Kunststoff gefertigt sein, wie beispielhaft Polyethylen, Polypropylen, Polyurethan oder einem Gemisch daraus. Alternativ oder zusätzlich kann der Schwimmkörper aus einem luftgefüllten Hohlkörper und/oder aus einem natürlichen Material gefertigt sein, wie Holz, Kork oder ein Gemisch daraus.

Alternativ zu den Einzelbauteilen können das Membranelement und der Schwimmkörper auch bevorzugt materialeinheitlich und/oder einstückig ausgeführt sein. In diesem Fall ist der Celluloseträger besonders kostengünstig herstellbar, da ein Fügeschritt zum Verbinden von Membranelement und Schwimmkörper entfallen kann.

Bevorzugt kann der Celluloseträger eine Celluloseträger-Unterseite und eine Celluloseträger-Oberseite aufweisen, wobei vorzugsweise die Celluloseträger-Unterseite dem Behälterboden des Behälters zugewandt ist und die Celluloseträger-Oberseite dem Behälterboden des Behälters abgewandt ist.

Besonders bevorzugt kann der Schwimmkörper Mittelstege aufweisen, die materialeinheitlicher und/oder einstückiger Bestandteil des Schwimmkörpers sein können. Bei einem großflächig dimensionierten Celluloseträger verhindern die Mittelstege, dass ein biegeschlaff ausgeführtes Membranelement nach unten, d.h. in Lotrichtung, ausbeult und das Membranelement so die Form einer im Wesentlichen ebenen Scheibe annimmt. Qualitätsschwankungen in der Cellulose-Schichtstruktur können so verhindert werden.

In einer konkreten Ausführungsform kann die Cellulose-Schichtstruktur, vorzugsweise ausschließlich und/oder unmittelbar, auf der Celluloseträger-Oberseite angeordnet sein. Alternativ oder zusätzlich kann die Cellulose-Schichtstruktur, vorzugsweise ausschließlich und/oder unmittelbar, auf der Membranelement-Oberseite angeordnet sein.

In einer bevorzugten Ausführungsform kann die Membranelement-Unterseite, vorzugsweise unmittelbar, mit der dem Kulturmedium oder einer Oberfläche des Kulturmediums in Kontakt stehen. Alternativ oder zusätzlich kann der Celluloseträger, vorzugsweise das Membranelement und/oder der Schwimmkörper, durchlässig für das Kulturmedium, vorzugsweise für das Nährmedium und/oder die cellulosebildenden Mikroorganismen, sein. Insbesondere dadurch, dass auch der Schwimmkörper für das Kulturmedium durchlässig ist, wird die Cellulose-Schichtstruktur auch im Bereich des Schwimmkörpers zuverlässig mit Nährmedium versorgt.

Aufgrund der Durchlässigkeit kann das Kulturmedium, vorzugsweise das Nährmedium und/oder die cellulosebildenden Mikroorganismen, ausgehend von der Membranelement-Unterseite bis zu der Membranelement-Oberseite durch das Membranelement diffundieren und/oder ausgehend von einer Schwimmkörper-Unterseite bis zu einer Schwimmkörper-Oberseite durch das Membranelement diffundieren. Der Celluloseträger stützt also nicht nur die Cellulose-Schichtstruktur und schützt diese vor dem Absinken in das Kulturmedium, sondern trägt auch dazu bei, dass das Bilden und/oder Wachstum der Cellulose-Schichtstruktur durch ausreichende Nährstoffversorgung sichergestellt ist.

Bevorzugt kann das Membranelement durch eine Mikrofiltrationsmembran oder durch eine Ultrafiltrationsmembran gebildet sein. Die Mikro- oder Ultrafiltrationsmembran kann zumindest teilweise oder ausschließlich aus Polyethersulfon und Keramik gefertigt sein. Alternativ dazu kann die Mikro- oder Ultrafiltrationsmembran ausschließlich aus Polyethersulfon oder ausschließlich aus Keramik gefertigt sein.

In einer besonders bevorzugten Ausführungsform kann das Membranelement durch ein textiles Gebilde, vorzugsweise durch ein textiles Flächengebilde, gebildet sein. Bevorzugt kann vorgesehen sein, dass das Membranelement durch ein Gewebe und/oder durch ein Gestrick und/oder durch ein Vlies, gebildet ist. Besonders bevorzugt kann vorgesehen sein, dass das textile Gebilde eine Porengröße in einem Bereich von 0,1 mm bis 3,5 mm, vorzugsweise in einem Bereich von 0,5 mm bis 1,5 mm aufweist. Das Membranelement als textiles Flächengebilde ist gut verfügbar und kostengünstig zu beschaffen.

Das textile Flächengebilde am Kontaktbereich von Kulturmedium und sauerstoffhaltigem Gas fördert das Wachstum der Cellulose-Schichtstruktur, so dass es im Vergleich zu einer Anordnung ohne Celluloseträger zu einer um 10% bis 20% erhöhten Celluloseproduktion kommt.

In einer konkreten Ausführungsform kann der Celluloseträger eine Celluloseträger-Außenumfangsfläche aufweisen, die an eine Behälterwand-Innenumfangsfläche der Behälterwand des Behälters konturangepasst sein kann. Alternativ oder zusätzlich kann der Celluloseträger eine Celluloseträger-Außenumfangsfläche aufweisen, mit der der Celluloseträger, in Umfangsrichtung des Celluloseträgers gesehen, vorzugsweise vollständig und/oder lückenlos in Gleitverbindung mit einer Behälterwand-Innenumfangsfläche der Behälterwand stehen kann. Bevorzugt kann vorgesehen sein, dass die Celluloseträger-Außenumfangsfläche ausschließlich in Gleitverbindung mit der Behälterwand-Innenumfangsfläche steht.

Beispielhaft kann die Celluloseträger-Außenumfangsfläche zumindest teilweise oder vollständig von einer Außenumfangsfläche des Membranelementes gebildet sein. Alternativ oder zusätzlich kann die Celluloseträger-Außenumfangsfläche zumindest teilweise oder vollständig von einer Außenumfangsfläche des Schwimmkörpers gebildet sein.

In einer besonders bevorzugten Ausführungsform kann das Kulturmedium, vorzugsweise vollständig und/oder ausschließlich, von der Behälterwand, dem Behälterboden und dem Celluloseträger umschlossen sein. Alternativ oder zusätzlich kann der Behälter undurchlässig für das Kulturmedium, vorzugsweise das Nährmedium und/oder die cellulosebildenden Bakterien, sein. Bevorzugt kann das Kulturmedium, vorzugsweise ausschließlich, über den Celluloseträger in Kontakt mit dem Gas stehen. Der Behälter, vorzugsweise die Behälterwand und/oder der Behälterboden, ist frei von Fluid-Öffnungen, durch die Kulturmedium in den Behälter ein oder aus dem Behälter ausströmen kann.

In einer konkreten Ausführungsform kann der Behälter, vorzugsweise die Behälterwand und/oder der Behälterboden, Fluid-Öffnungen aufweisen, durch die Kulturmedium in den Behälter ein oder aus dem Behälter ausströmen kann. Bevorzugt kann der Behälter in einen Fluid-Kreislauf eingebunden sein, wobei das Kulturmedium mittels der Fluid-Öffnungen, vorzugsweise kontinuierlich, durch den Behälter gepumpt wird.

Wie bereits erwähnt, wächst die Cellulose-Schichtstruktur entgegen der Lotrichtung Schicht für Schicht. Eine neu zu bildende Cellulose-Schicht der Cellulose-Schichtstruktur wird dadurch mit Kulturmedium, vorzugsweise Nährmedium, versorgt, dass das Kulturmedium, vorzugsweise das Nährmedium, durch das Membranelement und die zwischen der neu zu bildenden Cellulose-Schicht und dem Membranelement angeordneten, bereits gebildeten Cellulose-Schichten hindurch diffundiert. Mit zunehmender Schichtstrukturdicke der Cellulose-Schichtstruktur wird die Diffusion des Kulturmediums, vorzugsweise des Nährmediums, beeinträchtigt und auch verlangsamt, so dass die erreichbare Schichtstrukturdicke auf einen Bereich von 4 bis 6 cm begrenzt ist. Um höhere, vorzugsweise unbegrenzt hohe, Schichtstrukturdicken erreichen zu können und die Bildung der Cellulose-Schichtstruktur zu beschleunigen, kann bevorzugt ein Tropf- oder Sprühsystem vorgesehen sein. Mittels dem Tropf- oder Sprühsystem kann Kulturmedium oder Nährmedium von oben und in Lotrichtung auf die Cellulose-Schichtstruktur aufgetropft oder aufgesprüht werden und die zu bildende Cellulose-Schicht zusätzlich mit Kulturmedium oder Nährmedium versorgt werden.

Mittels des Tropf- oder Sprühsystems oder unabhängig davon können der Cellulose-Schichtstruktur auch Additive, vorzugsweise in festem oder flüssigen Aggregatszustand, zugeführt werden, die beispielsweise zu einer Wachstumsbeschleunigung der Cellulose-Schichtstruktur beitragen. Bevorzugt kann der Celluloseträger derart ausgebildet sein, dass der Celluloseträger auch dann zuverlässig auf dem Kulturmedium schwimmt, insofern über das Tropf- oder Sprühsystem Stoffe zugeführt werden, die das Gewicht der Cellulose-Schichtstruktur und/oder des Celluloseträgers erhöhen.

Erfindungsgemäß ist auch ein Celluloseträger für die vorbeschriebene Anordnung.

Ebenfalls erfindungsgemäß ist ein Verfahren zur Herstellung einer Cellulose-Schichtstruktur, vorzugsweise mit der vorbeschriebenen Anordnung, bei dem ein Behälter bereitgestellt wird, der zumindest teilweise mit einem cellulosebildenden Kulturmedium befüllt wird, das Nährmedium und cellulosebildende Mikroorgansimen aufweist. In den Behälter werden ferner, ein, vorzugsweise sauerstoffhaltiges, Gas und ein Celluloseträger eingebracht. Der Celluloseträger wird in Kontakt mit dem Kulturmedium oder in Kontakt mit einer Oberfläche des Kulturmediums gebracht, wobei sich durch Wechselwirkung der cellulosebildenden Mikroorganismen, des Kulturmediums und des Gases an dem Celluloseträger eine Cellulose-Schichtstruktur bildet. Der Celluloseträger ist als ein beweglicher und/oder bewegbarer Celluloseträger ausgebildet, der relativ zum Behälter beweglich ist.

Bevorzugt kann das Kulturmedium dadurch hergestellt werden, dass tiefgefrorene, cellulosebildende Mikroorganismen, vorzugsweise Bakterien, in einer Startkultur zunächst vermehrt und dann in das Nährmedium eingebracht werden. Alternativ können die cellulosebildenden Bakterien auch in dem Behälter vermehrt werden.

Besonders bevorzugt kann die hergestellte Cellulose-Schichtstruktur in einem Nachbehandlungsschritt gereinigt, getrocknet und/oder zur Eigenschaftsbeeinflussung der Cellulose-Schichtstruktur getränkt und mechanisch bearbeitet werden.

Nachfolgend sind Ausführungsbeispiele der Erfindung anhand der beigefügten Figuren beschrieben.

Es zeigen:
- Figur 1: in einer Seitenschnittansicht eine Anordnung zur Herstellung einer Cellulose-Schichtstruktur mit einem Celluloseträger;
- Figur 2: in einer Seitenschnittansicht den Celluloseträger in Alleinstellung;
- Figur 3: in einer Ansicht von oben den Celluloseträger;
- Figur 4: in einer Ansicht von unten den Celluloseträger mit einer den Celluloseträger umgebenden Behälterwand;
- Figur 5: in einer Ansicht von oben einen Celluloseträger mit Querstreben;
- Figur 6: in einer Ansicht von oben einen kreisscheibenförmigen Celluloseträger; und
- Figuren 7 bis 10: anhand von Seitenschnittansichten ein Verfahren zur Herstellung einer Cellulose-Schichtstruktur.

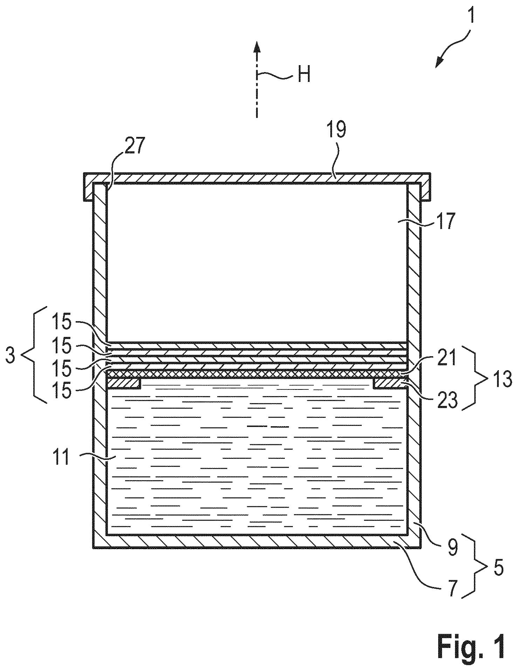

In der Figur 1 ist eine Anordnung 1 zur Herstellung einer Cellulose-Schichtstruktur 3 dargestellt. Die Anordnung 1 weist einen beispielhaft als Glasbehälter ausgebildeten Behälter 5 auf. Der Behälter 5 weist einen Behälterboden 7 und deine Behälterwand 9 auf und ist teilweise mit einem Kulturmedium 11 gefüllt. Das Kulturmedium 11 enthält flüssiges Nährmedium und cellulosebildende Bakterien der Art Komagataeibacter xylinus. Auf dem Kulturmedium 11 schwimmend und nicht starr, sondern in Gleitverbindung mit einer Behälterwand-Innenfläche stehend, ist ein Celluloseträger 13 angeordnet. Auf dem Celluloseträger 13 ist die Cellulose-Schichtstruktur 3 angeordnet, die aus mehreren, untereinander quervernetzten, Cellulose-Schichten 15 gebildet ist. Die Cellulose-Schichtstruktur 3 ist von gallertartige Konsistenz. In dem Behälter 5 befindet sich außerdem sauerstoffhaltiges Gas 17, das von den cellulosebildenden Bakterien unter Aufbrauch des Nährmediums zu Cellulose verstoffwechselt wird, die zu der auf dem Celluloseträger 13 angeordneten Cellulose-Schichtstruktur 3 wächst. Zur Vermeidung von Kontaminationen ist der Behälter 5 mit einem Deckel 19 verschlossen. Zur Sicherstellung einer ausreichenden Sauerstoffkonzentration ist der Deckel derart ausgestaltet, dass er für das sauerstoffhaltige Gas, vorzugsweise die Umgebungsluft, durchlässig ist.

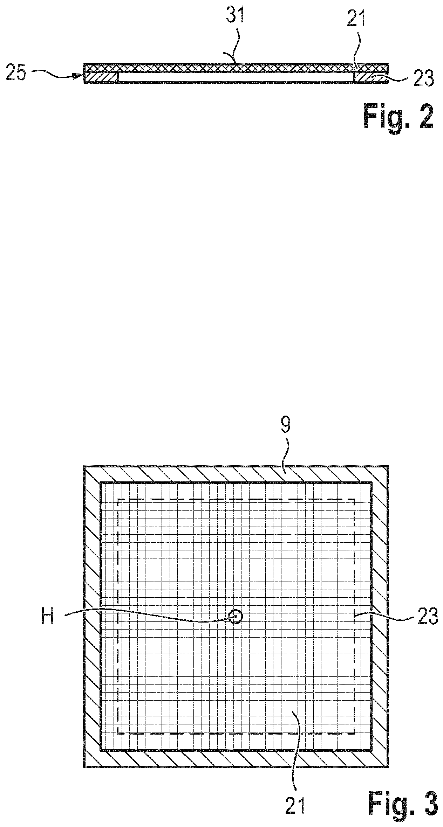

Wie in der Figur 2 dargestellt, weist der Celluloseträger 13 ein Membranelement 21 und einen mit dem Membranelement 21 verbundenen und aus einem einzelnen Schwimmkörperelement gebildeten Schwimmkörper 23 auf. Der Schwimmkörper 23 ist aus nichttoxischem, schwimmfähigen Material gefertigt, und zwar aus geschäumten Polyurethan. Der Schwimmkörper 23 weist eine Schwimmkörper-Oberseite auf, an der das Membranelement 21 befestigt ist. Das Membranelement 21 ist durch ein textiles Flächengebilde, und zwar durch Baumwollgewebe gebildet. Alternativ zu dem Baumwollgewebe kann das Membranelement 21 auch durch Ananasfaservlies gebildet sein. Sowohl das Material des Membranelementes 21 als auch das Material des Schwimmkörpers 23, das geschäumte Polyurethan, ist durchlässig für das sauerstoffhaltige Gas 17 und für das Kulturmedium 11, insbesondere durchlässig für das Nährmedium und/oder für die cellulosebildenden Bakterien.

In der Figur 3 ist der in den Behälter 5 eingesetzte Celluloseträger 13 in einer Ansicht von oben dargestellt. Der Celluloseträger 13 ist im Wesentlichen rechteckscheibenförmig ausgebildet und liegt mit einer Celluloseträger-Außenumfangsfläche 25 (siehe Fig. 2) vollflächig an einer Behälterwand-Innenumfangsfläche 27 (siehe Fig. 1) an. Das heißt, dass der Celluloseträger 13 das Kulturmedium 11 vollständig bedeckt.

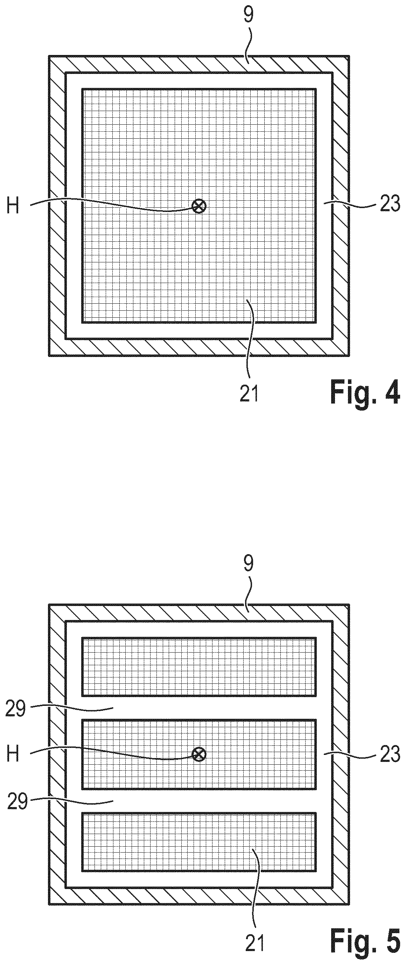

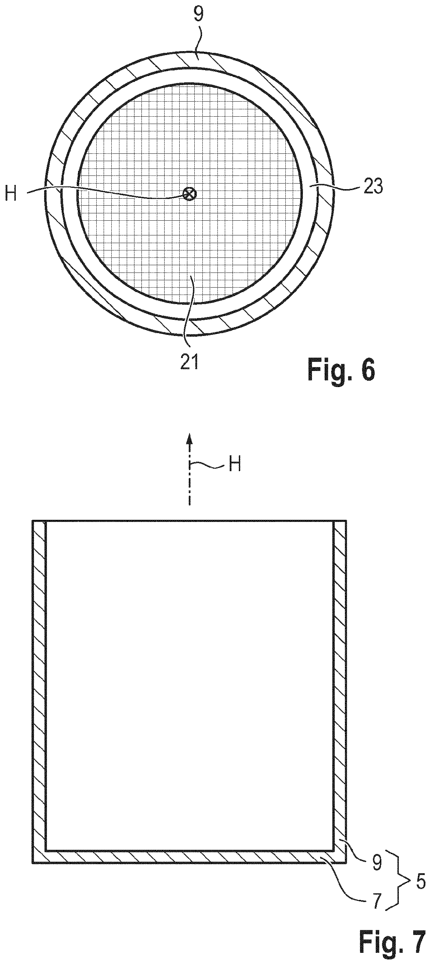

In der Figur 4 ist der in den Behälter 5 eingesetzte Celluloseträger 13 in einer Ansicht von unten dargestellt. Der Schwimmkörper 23 weist die Form einer rechteckförmigen Ringscheibe mit rechteckigem Querschnittsprofil auf und erstreckt sich entlang der Behälterwand-Innenumfangsfläche 27. In der Figur 5 weist der Celluloseträger 13 Mittelstege 29 auf, die sich unterhalb des Membranelementes 21 erstrecken und dieses stützen. Die Mittelstege 29 sind einstückiger und materialeinheitlicher Bestandteil des Schwimmkörpers 23. In der Figur 6 weist der Celluloseträger 13 die Form einer Kreisscheibe auf. Der Celluloseträger 13 in dieser Form kann insbesondere dann vorteilhaft eingesetzt werden, insofern der Behälter 5 eine hohlzylinderförmige Behälterwand 9 aufweist.

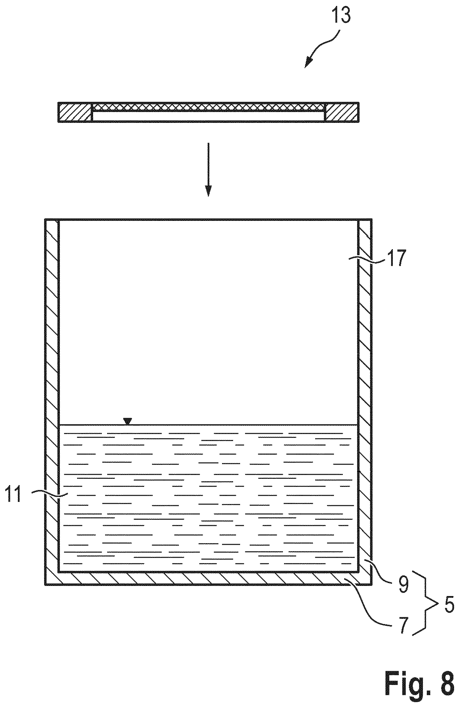

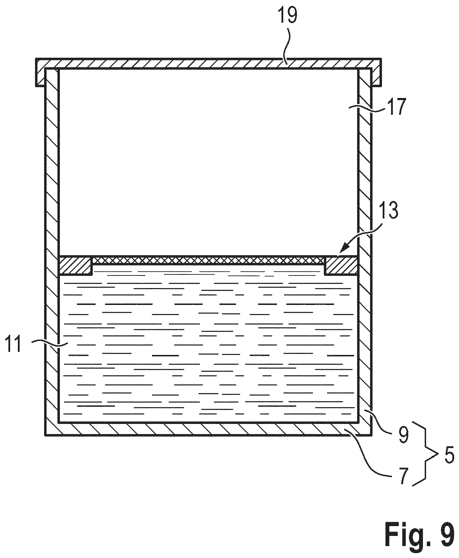

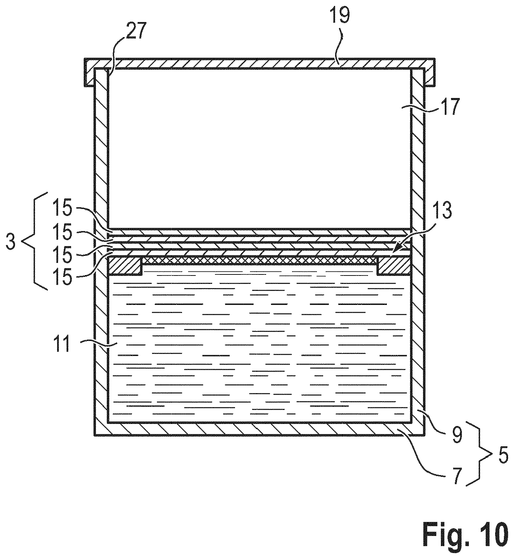

Anhand der Figuren 7 bis 10 sind einzelne Verfahrensschritte zur Herstellung der Cellulose-Schichtstruktur 3 veranschaulicht. Gemäß der Figur 7 wird bei dem Verfahren zunächst der nach oben offene Behälter 5 bereitgestellt. Entsprechend der Darstellung in der Figur 8 wird der nach oben offene Behälter 5 mit dem Kulturmedium 11 teilweise befüllt. Anschließend wird der Celluloseträger 13 in den Behälter 5 eingesetzt, der aufgrund des Auftriebes des Membranelementes und/oder des Schwimmkörpers 23 selbsttätig auf der Oberfläche des Kulturmediums 11 schwimmt. Daraufhin wird der Behälter 5 mit dem Deckel 19 geschlossen (siehe Figur 9), so dass vorzugsweise während der Herstellung der Cellulose-Schichtstruktur 3 keine Verunreinigungen, bspw. in Form von großen Pilzsporen oder Staub, in den Behälter gelangen können. Die Anordnung wird anschließend temperiert, und zwar auf eine Temperatur in einem Temperaturbereich von 25°C bis 35°C, vorzugsweise in einem Temperaturbereich von 28°C bis 30°C. Innerhalb von einer Zeitdauer von 5 Tagen bis 6 Wochen bildet sich auf dem Celluloseträger 13 die gallertartige Cellulose-Schichtstruktur 3 aus den einzelnen, untereinander quervernetzten, Cellulose-Schichten 15, aus, die mittels des Celluloseträger 13 am Absinken in das Kulturmedium 11 gehindert werden. Die Cellulose-Schichten 15 bilden sich ausgehend von einer Membranelement-Oberseite 31 des Membranelementes 21 schichtweise aus, und zwar in positiver Achsenrichtung der Behälter-Hochachse H (siehe Fig. 1) und somit entgegen der Lotrichtung. Die einzelnen Cellulose-Schichten 15 sind für das Kulturmedium 11 durchlässig, so dass das Kulturmedium 11, insbesondere das Nährmedium, durch die Cellulose-Schichten 15 diffundieren kann. Später gewachsene und damit oben liegende Cellulose-Schichten 15 werden also von Kulturmedium 11, insbesondere Nährmedium, versorgt, das sowohl durch das Membranelement 21 als auch durch die unten liegenden Cellulose-Schichten 15 diffundiert.

### Bezugszeichenliste

- 1: Anordnung
- 3: Cellulose-Schichtstruktur
- 5: Behälter
- 7: Behälterboden
- 9: Behälterwand
- 11: Kulturmedium
- 13: Celluloseträger
- 15: Cellulose-Schicht
- 17: Gas
- 19: Deckel
- 21: Membranelement
- 23: Schwimmkörper
- 25: Celluloseträger-Außenumfangsfläche
- 27: Behälterwand-Innenumfangsfläche
- 29: Mittelsteg
- 31: Membranelement-Oberseite
- H: Behälter-Hochachse

## Patentansprüche

1. Anordnung zur Herstellung einer Cellulose-Schichtstruktur,
mit einem Behälter (5), cellulosebildenden Mikroorganismen, einem Kulturmedium (11), einem Gas (17) und einem Celluloseträger (13),
wobei der Behälter (5) zumindest teilweise mit dem Kulturmedium (11) befüllt ist,
wobei der Celluloseträger (13) mit dem Kulturmedium (11) in Kontakt steht,
und wobei die Anordnung (1) dazu ausgebildet ist, dass sich durch Wechselwirkung der Mikroorganismen, des Kulturmediums (11) und des Gases (17) an dem Celluloseträger (13) eine Cellulose-Schichtstruktur (15) bildet,
**dadurch gekennzeichnet,**
**dass** der Celluloseträger (13) als ein relativ zum Behälter (5) beweglicher Celluloseträger (13) ausgebildet ist.

2. Anordnung nach Anspruch 1, **dadurch gekennzeichnet, dass** der Celluloseträger (13) als ein auf dem Kulturmedium (11) schwimmender Celluloseträger ausgebildet ist und/oder dass der Celluloseträger (13) auf dem Kulturmedium (11) schwimmt.

3. Anordnung nach Anspruch 1 oder 2, **dadurch gekennzeichnet, dass** der Celluloseträger (13) ein Membranelement (21) und einen Schwimmkörper (23) aufweist, wobei bevorzugt vorgesehen ist, dass der Celluloseträger (13), vorzugsweise das Membranelement (21) und/oder der Schwimmkörper (23), für das Kulturmedium (11), vorzugsweise für das Nährmedium und/oder für die cellulosebildenden Mikroorganismen, durchlässig ist, und/oder dass der Schwimmkörper (23), vorzugsweise in Bezug auf eine Behälter-Hochachse (H) gesehen, zumindest teilweise oder vollständig an und/oder unter einer Membranelement-Unterseite des Membranelementes (21) angeordnet ist.

4. Anordnung nach einem der vorhergehenden Ansprüche, **dadurch gekennzeichnet, dass** die Cellulose-Schichtstruktur (3), vorzugsweise ausschließlich und/oder unmittelbar, auf einer Celluloseträger-Oberseite des Celluloseträgers (13) angeordnet ist, und/oder dass die Cellulose-Schichtstruktur (3), vorzugsweise ausschließlich und/oder unmittelbar, auf einer Membranelement-Oberseite (31) des Membranelementes (21) angeordnet ist.

5. Anordnung nach einem der vorhergehenden Ansprüche, **dadurch gekennzeichnet, dass** eine Membranelement-Unterseite des Membranelementes (21), vorzugsweise unmittelbar, mit dem Kulturmedium (11) in Kontakt steht, und/oder dass der Celluloseträger (13), vorzugsweise das Membranelement (21) und/oder der Schwimmkörper (23), durchlässig für das Kulturmedium (11), vorzugsweise für das Nährmedium und/oder die cellulosebildenden Mikroorganismen, ist.

6. Anordnung nach einem der Ansprüche 3 bis 5, **dadurch gekennzeichnet, dass** das Membranelement (21) durch ein textiles Gebilde, vorzugsweise durch ein textiles Flächengebilde, gebildet ist, wobei bevorzugt vorgesehen ist, dass das Membranelement (21) durch ein Gewebe und/oder durch ein Gestrick und/oder durch ein Vlies, gebildet ist, wobei besonders bevorzugt vorgesehen ist, dass das textile Gebilde eine Porengröße in einem Bereich von 0,1 mm bis 3,5 mm, vorzugsweise in einem Bereich von 0,5 mm bis 1,5 mm aufweist.

7. Anordnung nach einem der vorhergehenden Ansprüche, **dadurch gekennzeichnet, dass** der Celluloseträger (13) eine Celluloseträger-Außenumfangsfläche (25) aufweist, die an eine Behälterwand-Innenumfangsfläche (27) einer Behälterwand (9) des Behälters (5) konturangepasst ist, und/oder dass der Celluloseträger (13) eine Celluloseträger-Außenumfangsfläche (25) aufweist, mit der der Celluloseträger (13), in Umfangsrichtung des Celluloseträgers (13) gesehen, vollständig und/oder lückenlos in Gleitverbindung mit der Behälterwand-Innenumfangsfläche (27) steht.

8. Anordnung nach einem der vorhergehenden Ansprüche, **dadurch gekennzeichnet, dass** der Behälter (5) eine Behälterwand (9) und einen Behälterboden (7) aufweist, und dass das Kulturmedium (11), vorzugsweise vollständig und/oder ausschließlich, von der Behälterwand (9), dem Behälterboden (7) und dem Celluloseträger (13) umschlossen ist, und/oder dass der Behälter (5) undurchlässig für das Kulturmedium (11), vorzugsweise das Nährmedium und/oder die cellulosebildenden Bakterien, ist, und/oder dass das Kulturmedium (11), vorzugsweise ausschließlich, über den Celluloseträger (13) in Kontakt mit dem Gas (17) steht, und/oder dass der Behälter (5), vorzugsweise die Behälterwand (9) und/oder der Behälterboden, Fluid-Öffnungen aufweist, mittels derer das Kulturmedium (11) in den Behälter (5), vorzugsweise kontinuierlich, ein- und/oder aus dem Behälter (5) ausleitbar ist.

9. Celluloseträger für eine Anordnung nach einem der vorhergehenden Ansprüche.

10. Verfahren zur Herstellung einer Cellulose-Schichtstruktur, vorzugsweise mit einer Anordnung nach einem der Ansprüche 1 bis 8.
